# FASCICULE DE BREVET EUROPEEN

(11) **EP 2 135 670 B1**
(45) Date de publication et mention de la délivrance du brevet: **01.03.2017**
(21) Numéro de dépôt: 09290405.1
(22) Date de dépôt: 29.05.2009
(51) Int. Cl.: B01J 21/04, B01J 23/44, B01J 37/00, B01J 37/02, B01J 37/10, C10G 45/40, C01F 7/02, C07C 5/02

(54) **Catalyseur d'hydrogénation sélective**
Katalysator zur selektiven Hydrierung
A selective hydrogenation catalyst

(30) Priorité: 20.06.2008 FR 0803482
(43) Date de publication de la demande: 23.12.2009
(73) Titulaire: IFP Énergies nouvelles, 92852 Rueil-Malmaison Cedex (FR)
(72) Inventeur: Fecant, Antoine, 69530 Brignais (FR); Fischer, Lars, 38200 Vienne (FR); Rebours, Bernadette, 69007 Lyon (FR); Revel, Renaud, 38200 Serpaize (FR); Thomazeau, Cécile, 69530 Brignais (FR)

(56) Documents cités:
- EP-A- 0 576 828
- EP-A- 0 903 177
- EP-A- 1 897 613
- FR-A- 2 787 040
- US-B1- 6 437 206

## Description

Le procédé d'hydrogénation sélective permet de transformer les composés polyinsaturés des coupes pétrolières par conversion des composés les plus insaturés vers les alcènes correspondants en évitant la saturation totale et donc la formation des alcanes correspondants.

L'objet de l'invention est de proposer un catalyseur à performances améliorées appliqué au procédé d'hydrogénation sélective des composés hydrocarbonés insaturés présents dans les coupes d'hydrocarbures, de préférence des coupes issues du vapocraquage ou du craquage catalytique.

Dans la suite, les groupes d'éléments chimiques sont donnés selon la classification CAS (CRC Handbook of Chemistry and Physics, éditeur CRC press, rédacteur en chef D.R. Lide , 81ème édition, 2000-2001). Par exemple, le groupe VIII selon la classification CAS correspond aux métaux des colonnes 8, 9 et 10 selon la nouvelle classification IUPAC.

### ART ANTERIEUR

Les catalyseurs d'hydrogénation sélective sont généralement à base de métaux du groupe VIII du tableau périodique des éléments, de préférence le palladium ou le nickel. La phase active des catalyseurs se présente sous la forme de petites particules métalliques déposées sur un support qui peut être un oxyde réfractaire sous forme de billes, d'extrudés, de trilobes ou sous des formes présentant d'autres géométries. La teneur en métal, la présence éventuelle d'un deuxième élément métallique, la taille des particules de métal et la répartition de la phase active dans le support font partie des critères qui ont une importance sur l'activité et la sélectivité des catalyseurs.

La présente invention vise à obtenir des catalyseurs à base de palladium ayant des propriétés physico-chimiques et des performances catalytiques améliorées par rapport aux catalyseurs à base de palladium de l'art antérieur.

Les catalyseurs selon l'invention comprennent du palladium déposé sur un support d'alumine, ledit support d'alumine possédant une structure cristallographique et donc un diffractogramme particulier obtenu par diffraction de rayons X. Cette propriété particulière du support est à l'origine de l'amélioration des performances catalytiques.

Des brevets de l'art antérieur présentent des catalyseurs d'hydrogénation sélective comprenant au moins un métal noble et un support à base d'alumine, ou des supports de catalyseurs de type oxyde d'aluminium, dont les diffractogrammes de rayons X sont particuliers. On peut notamment citer les brevets US6437206B et EP0576828. Cependant, ces diffractogrammes obtenus pour les supports ou les catalyseurs selon l'art antérieur diffèrent de ceux selon la présente invention par la présence et/ou l'absence et/ou l'intensité des pics obtenus pour des distances interréticulaires données.

### RESUME DE L'INVENTION

L'invention concerne un catalyseur comprenant du palladium sur un support oxyde d'aluminium. Le support d'oxyde d'aluminium présente à l'état calciné avant imprégnation du palladium un diffractogramme obtenu par diffraction de rayons X enregistrée sur un diffractomètre en géométrie Bragg-Brentano équipé d'un tube de cuivre (1.54 Å) comprenant des raies qui correspondent aux distances interréticulaires et aux intensités relatives suivantes :

| Distances interréticulaires d (10⁻¹⁰ m) +/- 5.10⁻³d | Intensités relatives I/Io (en %) |
|---|---|
| 4,54 | 3-10 |
| 2,70-2,75 | 5-25 |
| 2,41 | 35-45 |
| 2,28 | 15-30 |
| 2,10 | 0-10 |
| 1,987 | 30-50 |
| 1,958 | 30-50 |
| 1,642 | 0-5 |
| 1,519 | 10-20 |
| 1,394 | 100 |

lesdites distances interréticulaires et intensités relatives étant déterminées par modélisation complète à l'aide de fonctions analytiques de type pseudo-Voigt symétriques, de rapport Gaussienne-Lorentzienne fixé à 0,5,
et dans lequel la teneur en palladium dans le catalyseur est comprise entre 0,03 et 1 % poids et la surface spécifique du support est comprise entre 100 et 160 m²/g.

### DESCRIPTION DETAILLEE DE L'INVENTION

La présente invention vise à obtenir des catalyseurs à base de palladium ayant des propriétés physico-chimiques et des performances catalytiques améliorées par rapport aux catalyseurs à base de palladium de l'art antérieur. Plus précisément, la présente invention propose un procédé de préparation d'un support alumine ainsi que la préparation d'un catalyseur sur ce support alumine, et un procédé d'hydrogénation sélective mettant en oeuvre ledit catalyseur conduisant à des performances catalytiques améliorées dudit catalyseur.

La présente invention concerne des catalyseurs comprenant du palladium sur un support alumine. Le support alumine présente à l'état calciné un diffractogramme obtenu par diffraction de rayons X comprenant des raies qui correspondent aux distances interréticulaires et aux intensités relatives suivantes :

| Distances interréticulaires d (10⁻¹⁰ m) +/- 5.10⁻³d | Intensités relatives I/Io (en %) |
|---|---|
| 4,54 | 3-10 |
| 2,70-2,75 | 5-25 |
| 2,41 | 35-45 |
| 2,28 | 15-30 |
| 2,10 | 0-10 |
| 1,987 | 30-50 |
| 1,958 | 30-50 |
| 1,642 | 0-5 |
| 1,519 | 10-20 |
| 1,394 | 100 |

Seules les raies dont l'intensité relative est supérieure ou égale à 1% sont considérées.

Dans tout le texte, les distances interréticulaires sont données avec une précision relative de 0,5% notée +/- 5.10⁻³d.

Le diffractogramme est caractéristique de la structure spécifique du support du catalyseur selon l'invention. Le support alumine peut comprendre des impuretés et des additifs tant que le diffractogramme reste tel que décrit ci dessus. Par exemple, le support peut comprendre des oxydes inorganiques tels que les oxydes de métaux des groupes IIA, IIIB, IVB, IIB, IIIA, IVA selon la classification CAS, de préférence la silice, le dioxyde de titane, le dioxyde de zirconium, l'oxyde de zinc, l'oxyde de magnésium et l'oxyde de calcium.

La teneur en cation des colonnes IIA, IIIB, IVB, IIB, IIIA, IVA est de préférence comprise entre 0,01 et 30 % en poids, de préférence entre 0,01 et 10 % en poids, de façon encore plus préférée entre 0,03 et 1,5% en poids. La teneur en un ou plusieurs métaux du groupe IB peut être comprise entre 1 à 10 000 ppm poids par rapport au support.

La teneur maximale en oxydes autres que l'alumine dans le support dépend des oxydes présents. Elle peut être déterminée par le diffractogramme puisqu'à un changement de structure est associé un changement de diffractogramme. En général, la teneur en tels oxydes sera inférieure à 50%, de préférence inférieure à 30%, de manière très préférée entre 0,01% et 15% poids par rapport à la masse du support.

Selon l'invention, le support poreux se présente avantageusement sous forme de billes, de trilobes, d'extrudés, de pastilles, ou d'agglomérats irréguliers et non sphériques dont la forme spécifique peut résulter d'une étape de concassage. De manière très avantageuse, ledit support se présente sous forme de billes ou d'extrudés. De manière encore plus avantageuse, ledit support se présente sous forme de billes.

Le volume poreux du support est généralement compris entre 0,1 et 2,0 cm³/g, de préférence compris entre 0,5 et 1,5 cm³/g.

La surface spécifique du support poreux est comprise entre 100 et 160 m²/g.

La teneur en palladium dans le catalyseur est comprise entre 0,03 et 1 % poids et de manière encore plus préférée entre 0,05% et 0,8%.

Le diffractogramme du catalyseur sous forme oxyde peut donc présenter, en plus des raies caractéristiques du support, les raies caractéristiques du palladium, sous forme métal ou oxyde, ou sous une forme où il se trouve en combinaison avec d'autres composés (nitrate de palladium, chlorate de palladium, formiate de palladium). L'homme du métier se reportera aux tables ICDD (International Center for Diffraction Data) pour connaître les positions de ces raies, pour chacun des composés nommé ci dessus. Par exemple, les positions des raies de l'oxyde de palladium (PdO) sont reportées dans la table 00-041-1107, et les positions des raies du palladium métallique (Pd⁰) sont reportées dans la table 00-046-1043.

Dans le tableau ci-dessous, seules les raies d'intensités relatives I/I₀ supérieures à 10 et de distances interréticulaires supérieures à 1,35 ont été reportées. La colonne hkl correspond aux indices de Miller.

| PDF 41-1107 (PdO) | | | PDF 46-1043 (Pd⁰) | | |
|---|---|---|---|---|---|
| d(Ǻ). | I/I₀ | hkl | d(Ǻ) | I/I₀ | hkl |
| 2,67 | 22 | 002 | | | |
| 2,65 | 100 | 101 | | | |
| | | | 2,25 | 100 | 111 |
| 2.15 | 14 | 110 | | | |
| | | | 1,945 | 60 | 200 |
| 1,676 | 20 | 112 | | | |
| 1,535 | 15 | 103 | | | |
| 1,523 | 11 | 200 | | | |
| | | | 1,375 | 42 | 220 |

### PREPARATION DU SUPPORT

Selon une première variante les supports selon l'invention sont des agglomérés d'alumine se présentant sous forme de billes. Selon cette première variante la préparation du support comprend les étapes suivantes:
s1) déshydratation par calcination flash d'un hydroxyde ou d'un oxyhydroxyde d'aluminium, préférentiellement de l'hydrargillite, pour obtenir une poudre d'alumine active ;
   La calcination flash est un chauffage intense et rapide qui conduit à une déshydratation brutale d'un hydroxyde d'aluminium (hydrargillite, gibbsite ou bayerite) ou d'un oxyhydroxyde d'aluminium (boehmite ou diaspore), à l'aide d'un courant de gaz chaud permettant d'éliminer et d'entraîner très rapidement l'eau évaporée. La température est comprise entre 400 et 1200°C, de préférence entre 600°C et 900°C et la durée est comprise entre une fraction de seconde et 5 secondes, de préférence entre 0,1 seconde et 4 secondes. Comme composé de départ, on utilise préférentiellement de l'hydrargillite. L'expérience montre que ce composé est le plus favorable pour l'obtention d'un produit final ayant les propriétés recherchées. De plus, il est relativement bon marché.
   Généralement, la poudre d'alumine active obtenue après la déshydratation de l'hydroxyde ou oxyhydroxyde d'aluminium est broyée.
   Généralement, la poudre d'alumine active obtenue après la déshydratation de l'hydroxyde ou de l'oxyhydroxyde d'aluminium est lavée avec de l'eau ou une solution aqueuse acide.
s2) mise en forme de ladite poudre d'alumine active de manière à obtenir des billes d'une densité de remplissage en cru comprise entre 500 et 1100 kg/m3, préférentiellement incluse entre 700 et 950 kg/m3, et d'un diamètre compris majoritairement entre 0,8 et 10 mm, préférentiellement entre 1 et 5 mm ;
   La mise en forme de ladite poudre d'alumine active de manière à obtenir des billes, nommée granulation, est généralement réalisée au moyen d'une technologie tournante comme un drageoir tournant ou un tambour tournant. Ce type de procédé permet d'obtenir des billes de diamètre et de répartitions de pores contrôlées, ces dimensions et ces répartitions étant, en général, créées pendant l'étape d'agglomération. La porosité peut être créée par différents moyens, comme le choix de la granulométrie de la poudre d'alumine ou l'agglomération de plusieurs poudres d'alumine de différentes granulométries. Une autre méthode consiste à mélanger à la poudre d'alumine, avant ou pendant l'étape d'agglomération, un ou des composés, appelés porogènes, disparaissant par chauffage et créant ainsi une porosité dans les billes. Comme composés porogènes utilisés, on peut citer, à titre d'exemple, la farine de bois, le charbon de bois, le soufre, des goudrons, des matières plastiques ou émulsions de matières plastiques telles que le polychlorure de vinyle, des alcools polyvinyliques, la naphtaline ou analogues. La quantité de composés porogènes ajoutés est déterminée par le volume désiré.
   Lors de la mise en forme de ladite poudre d'alumine, on lui ajoute généralement un ou des matériaux porogènes disparaissant par chauffage. Lesdits matériaux porogènes sont sélectionnés dans le groupe constitué par la farine de bois, le charbon de bois, le soufre, les goudrons, les matières plastiques, les émulsions de matières plastiques, les alcools polyvinyliques et la naphtaline.
s3) traitement thermique à une température comprise entre 200 et 1200°C, de préférence entre 400 et 900°C desdites billes de manière à leur procurer une surface spécifique comprise entre 50 et 420 m²/g,
s4) traitement hydrothermal desdites billes par imprégnation avec de l'eau ou une solution aqueuse préférentiellement acide, puis séjour dans un autoclave à une température comprise entre 100 et 300°C, de préférence entre 150 et 250°C.
   Le traitement hydrothermal est généralement conduit à une température de 100 à 300°C, préférentiellement de 150 à 250°C, pendant une durée supérieure à 45 minutes, préférentiellement de 1 à 24 heures, très préférentiellement de 1,5 à 12 heures. Le traitement hydrothermal est généralement effectué à l'aide d'une solution aqueuse acide comprenant un ou plusieurs acides minéraux et/ou organiques de préférence l'acide nitrique, l'acide chlorhydrique, l'acide perchlorique, l'acide sulfurique, les acides faibles dont la solution a un pH inférieur à 4 comme l'acide acétique ou l'acide formique. Généralement, ladite solution aqueuse acide comprend également un ou plusieurs composés pouvant libérer des anions capables de se combiner avec les ions aluminium, de préférence les composés comprenant un ion nitrate (comme le nitrate d'aluminium), chlorure, sulfate, perchlorate, chloroacétate, trichloroacétate, bromoacétate, dibromoacétate, et les anions de formule générale : R-COO comme les formiates et les acétates.
s5) calcination des agglomérés ainsi obtenus à une température comprise entre 500 et 820°C, préférentiellement entre 550°C et 750°C.

Cette calcination est généralement réalisée afin d'obtenir une surface spécifique du support comprise entre 60 et 210 m²/g et d'obtenir le diffractogramme de rayons X désiré.

Selon une deuxième variante les supports selon l'invention sont des agglomérés d'alumine se présentant sous forme de matériaux extrudés. Selon cette deuxième variante la préparation du support comprend les étapes suivantes:
s1) malaxage et extrusion d'un matériau à base d'alumine pour le mettre en forme ;
   Généralement, ledit matériau à base d'alumine est de l'hydrargillite déshydratée. Le matériau à base d'alumine peut aussi généralement être issu de la précipitation de boehmite, pseudo-boehmite ou de bayerite, ou d'un mélange de tels matériaux.
   Lors de la mise en forme dudit matériau à base d'alumine, on lui ajoute généralement un ou des matériaux porogènes disparaissant par chauffage. Lesdits matériaux porogènes sont sélectionnés dans le groupe constitué par la farine de bois, le charbon de bois, le soufre, les goudrons, les matières plastiques, les émulsions de matières plastiques, les alcools polyvinyliques et la naphtaline.
s2) traitement thermique à une température comprise entre 200 et 1200°C des matériaux extrudés ainsi obtenus, de manière à leur procurer une surface spécifique comprise entre 50 et 420 m²/g ;
s3) traitement hydrothermal desdits matériaux extrudés, par imprégnation avec de l'eau ou une solution aqueuse préférentiellement acide, puis séjour dans un autoclave à une température comprise entre 100 et 300°C, de préférence entre 150 et 250°C.
   Le traitement hydrothermal est généralement conduit à une température de 100 à 300°C, préférentiellement de 150 à 250°C, pendant une durée supérieure à 45 minutes, préférentiellement de 1 à 24 heures, très préférentiellement de 1,5 à 12 heures. Le traitement hydrothermal est généralement effectué à l'aide d'une solution aqueuse acide comprenant un ou plusieurs acides minéraux et/ou organiques de préférence l'acide nitrique, l'acide chlorhydrique, l'acide perchlorique, l'acide sulfurique, les acides faibles dont la solution a un pH inférieur à 4 comme l'acide acétique ou l'acide formique. Généralement, ladite solution aqueuse acide comprend également un ou plusieurs composés pouvant libérer des anions capables de se combiner avec les ions aluminium, de préférence les composés comprenant un ion nitrate (comme le nitrate d'aluminium), chlorure, sulfate, perchlorate, chloroacétate, trichloroacétate, bromoacétate, dibromoacétate, et les anions de formule générale : R-COO comme les formiates et les acétates.
s4) calcination des agglomérés ainsi obtenus à une température comprise entre 500 et 820°C, préférentiellement entre 550°C et 750°C.

Cette calcination est généralement réalisée afin d'obtenir une surface spécifique du support comprise entre 60 et 210 m²/g et d'obtenir le diffractogramme de rayons X désiré.

### PREPARATION DES CATALYSEURS

Les catalyseurs sont préparés par toute méthode connue de l'homme du métier.

### c1) Préparation d'une solution comprenant le palladium

Le sel précurseur du palladium est généralement sélectionné dans le groupe constitué par le chlorure de palladium et le nitrate de palladium et le sulfate de palladium. De manière très préférée, le sel précurseur du palladium est le nitrate de palladium. La concentration de la solution aqueuse de précurseur de palladium est ajustée selon la teneur massique en palladium voulue sur le catalyseur.

Éventuellement, ladite solution de précurseur de palladium peut-être neutralisée par un hydroxyde sélectionné dans le groupe constitué par les hydroxydes d'alcalins et les hydroxydes d'alcalino-terreux pour former une suspension colloïdale de particules d'oxyde de palladium, le pH de ladite suspension colloïdale étant compris entre 1,0 et 3,5.

### c2) Imprégnation de la solution sur le support alumine

L'imprégnation du support alumine peut être réalisée par imprégnation à sec, en excès ou en défaut, en mode statique ou dynamique. L'imprégnation peut être réalisée en une ou plusieurs imprégnations successives.

### c3) Séchage du catalyseur

Le catalyseur imprégné est généralement séché afin d'éliminer toute ou une partie de l'eau introduite lors de l'imprégnation, de préférence à une température comprise entre 50 et 250°C, de manière plus préférée entre 70°C et 200°C. Le séchage est effectué sous air, ou sous atmosphère inerte (azote par exemple).

### c4) Calcination du catalyseur (étape optionnelle)

Le catalyseur est ensuite calciné, généralement sous air. La température de calcination est généralement comprise entre 300°C et 500°C. La durée de calcination est généralement comprise entre 0,5 heures et 5 heures.

### c5) Activation par réduction du catalyseur obtenu à l'étape précédente

Le catalyseur est généralement réduit. Cette étape est de préférence réalisée en présence d'un gaz réducteur, de préférence au moyen d'hydrogène gazeux in-situ, c'est-à-dire dans le réacteur où est réalisée la transformation catalytique.

De manière préférée, pour les catalyseurs à base de palladium, cette étape est effectuée à une température comprise entre 50°C et 300°C, de manière encore plus préférée entre 80°C et 160°C.

Selon une variante de préparation du catalyseur, le catalyseur est préparé en plusieurs imprégnations.

Pour les catalyseurs préparés en deux imprégnations, les enchaînements peuvent être les suivants :
- Imprégnation n°1 - Séchage - Imprégnation n°2 - Séchage - Calcination
- Imprégnation n°1 - Séchage - Calcination - Imprégnation n°2 - Séchage - Calcination

L'invention concerne aussi le catalyseur obtenu à partir des procédés de préparation de catalyseur décrits dans la présente invention.

### CARACTERISATION DU SUPPORT

Les diagrammes des différents supports cités dans ce document ont été enregistrés sur un diffractomètre (X'PERT'Pro de PANalytical) en géométrie Bragg-Brentano, équipé d'un tube de cuivre (1,54 Ǻ), d'un compteur proportionnel et de fentes à ouverture variable en fonction de 2θ. La surface d'échantillon irradiée était de 10x10mm, le pas d'échantillonnage de 0,05°2θ, le temps par pas de 5 à 15s.

Après enregistrement, les intensités ont été corrigées et transformées en intensités à volume irradié constant.

La mesure des positions, intensités relatives et largeurs des raies diffractées ont été déterminées par modélisation complète des diffractogrammes à l'aide de fonctions analytiques de type pseudo-Voigt symétriques, de rapport Gaussienne-Lorentzienne fixé à 0,5. Ces fonctions sont symétriques pour toutes les raies sauf pour la raie d'intensité maximale (d=1,39Ǻ) dont les deux demi largeurs ne sont pas équivalentes. Par ailleurs, pour décomposer le doublet de raies situées à 1,99 et 1,96 Ǻ, les deux fonctions pseudo-Voigt symétriques ont été contraintes à adopter la même largeur à mi hauteur.

Les positions, les intensités et les largeurs des fonctions ont été affinées pour ajuster les profils calculés aux raies expérimentales, nous avons affiné les positions, les intensités et les largeurs.

Les paramètres affinés des raies calculées qualifient les raies expérimentales :
o positions (distances interréticulaires)
o intensité
o FWHM (*full width at half the maximum,* largeur à mi-hauteur)

Un fond de diffusion linéaire a été ajusté en même temps que les profils de raies. Une raie extrêmement large et peu intense, centrée vers 2,5Ǻ, indispensable à la qualité de l'affinement du diagramme expérimental mais manifestement non définie, a été considérée comme un supplément de fond de diffusion.

Les intensités relatives reportées ici sont exprimées en pourcentage de la hauteur de la raie la plus intense (d=1,39Ǻ), au dessus du fond de diffusion.

Dans le cas du support selon l'invention, la raie la plus intense (d=1,39Ǻ) présente une dissymétrie que nous avons caractérisée par un "taux d'asymétrie" correspondant au rapport de la largeur gauche de la raie (en °2θ) sur sa largeur droite (en °2θ).

Les largeurs de raies sont qualifiées (fine (F), normale (N), large (L), très large (TL)) selon la gamme de largeur à mi hauteur (FWHM) du profil calculé pour chaque raie expérimentale. Ces largeurs sont dépendantes de l'appareillage et de la longueur d'onde utilisée. Les largeurs exprimées en 2θ sont dépendantes de l'appareillage et de la longueur d'onde utilisée pour l'analyse. Par contre, la qualification des raies (fine (F), normale (N), large (L), très large (TL)) déduite de ces valeurs est valable quel que soit le type d'appareil et les conditions d'analyse utilisées. Le classement relatif des raies, par rapport à leur largeur à mi-hauteur, les unes par rapport aux autres est valable quel que soit le type d'appareil et les conditions d'analyse utilisées.

La qualification des largeurs de raies est donnée ci dessous :

| Largeur de raie mesurée à mi-hauteur, dans nos conditions d'analyse (en degré 2θ) | Qualification de la largeur de raies |
|---|---|
| <0,8 | Fine (F) |
| 0,8 - 2,0 | Normale (N) |
| 2,0 - 3,0 | Large (L) |
| > 3,0 | Très Large (TL) |

Pour les catalyseurs selon l'invention comprenant du palladium sur un support oxyde d'aluminium, le support d'oxyde d'aluminium présente généralement à l'état calciné avant imprégnation du palladium un diffractogramme obtenu par diffraction des rayons X enregistrée sur un diffractomètre en géométrie Bragg-Brentano équipé d'un tube de cuivre (1.54 Ǻ) comprenant des raies qui correspondent aux distances interréticulaires, aux intensités relatives et aux largeurs de raie suivantes :

| Distances interréticulaires d (10⁻¹⁰ m) +/- 5.10⁻³d | Intensités relatives I/Io (en %) | Largeur de raie |
|---|---|---|
| 4,54 | 3-10 | L-TL |
| 2,70-2,75 | 5-25 | TL |
| 2,41 | 35-45 | L |
| 2,28 | 15-30 | F |
| 2,10 | 0-10 | N-TL |
| 1,987 | 30-50 | N |
| 1,958 | 30-50 | N |
| 1,642 | 0-5 | L-TL |
| 1,519 | 10-20 | TL |
| 1,394 | 100 | N |

Le rapport d'intensité des raies situées à 1.987 Ǻ et 1.958 Ǻ (I/I₀)_{1,987}/(I/l₀)_{1,958} est compris entre 1,1 et 1,8, de préférence entre 1,2 et 1,7.
Le taux d'asymétrie de la raie située à 1,394 Ǻ est compris entre 1,35 et 2,0.

### UTILISATION DU CATALYSEUR SELON L'INVENTION

Le catalyseur selon l'invention peut être utilisé dans les procédés faisant intervenir une transformation de composés organiques. Ainsi, le catalyseur selon l'invention peut être utilisé dans les procédés comprenant des réactions d'hydrogénation des composés comportant des fonctions aromatiques, cétones, aldéhydes, acides ou nitro, l'hydrogénation du monoxyde de carbone en alcools C1-C6, en méthanol ou en diméthyl-éther, les réactions d'isomérisation ou d'hydro-isomérisation, d'hydrogénolyse, et d'une manière générale les réactions faisant intervenir des coupures ou des formations de liaisons carbone-carbone.

Les conditions opératoires généralement utilisées pour ces réactions sont les suivantes : une température comprise entre 0°C et 500°C, de préférence entre 25 et 350°C, une pression comprise entre 0,1 et 20 MPa, de préférence entre 0,1 et 10 MPa, une vitesse volumique horaire (V.V.H.) comprise entre 0,1 et 50 h⁻¹, de préférence entre 0,5 et 20 h⁻¹ pour une charge liquide; et entre 500 et 30 000 h⁻¹, de préférence entre 500 et 15 000 h⁻¹ pour une charge gazeuse. Lorsque de l'hydrogène est présent, le rapport molaire hydrogène sur charge est compris entre 1 et 500 litres par litre, de préférence entre 10 et 250 litres par litre.

La mise en oeuvre du catalyseur selon l'invention et les conditions de son utilisation doivent être adaptées par l'utilisateur à la réaction et à la technologie utilisée.

Le catalyseur selon l'invention peut aussi être utilisé dans les réactions d'hydrogénation des composés comportant des fonctions acétyléniques, diéniques, oléfiniques.

L'invention concerne aussi le procédé d'hydrogénation sélective par mise en contact d'une charge sur le catalyseur selon l'invention ou sur le catalyseur préparé selon l'invention, ladite charge étant sélectionnée dans le groupe constitué par les coupes C3 de vapocraquage, les coupes C4 de vapocraquage, les coupes C5 de vapocraquage et les essences de vapocraquage appelée aussi essences de pyrolyse.

Selon une application préférée, les catalyseurs selon l'invention sont mis en oeuvre pour les réactions d'hydrogénation sélective des coupes poly-insaturées d'hydrocarbures issues de vapocraquage et/ou de craquage catalytique, de préférence des coupes poly-insaturées d'hydrocarbures issues du vapocraquage.

### Hydrogénation des coupes C3 à C5

Les procédés de conversion des hydrocarbures tels que le vapocraquage ou le craquage catalytique sont opérés à haute température et produisent une grande variété de molécules insaturées telles que l'éthylène, le propylène, les butènes linéaires, l'isobutène, les pentènes ainsi que des molécules insaturées contenant jusqu'à environ 15 atomes de carbone.

En parallèle sont également formés des composés polyinsaturés : acétylène, propadiène et méthylacétylène (ou propyne), 1-2 et 1-3 butadiène, vinylacétylène et éthylacétylène, et d'autres composés polyinsaturés dont le point d'ébullition correspond à la fraction essence C5⁺.

Tous ces composés polyinsaturés doivent être éliminés pour permettre l'utilisation de ces différentes coupes dans les procédés de pétrochimie tels que les unités de polymérisation.

Ainsi, par exemple, la coupe C3 de vapocraquage peut avoir la composition moyenne suivante : de l'ordre de 90% poids propylène, de l'ordre de 3 à 8% poids de propadiène et méthylacétylène, le reste étant essentiellement du propane. Dans certaines coupes C3, entre 0,1 et 2% poids de C2 et de C4 peut aussi être présent. Les spécifications concernant les concentrations de ces composés polyinsaturés pour les unités de pétrochimie et de polymérisation sont très basses : 20-30 ppm poids de MAPD (MéthylAcétylène et PropaDiène) pour le propylène qualité chimique et moins de 10 ppm poids voire jusqu'à 1 ppm poids pour la qualité « polymérisation ».

Une coupe C4 de vapocraquage présente par exemple la composition molaire moyenne suivante : 1 % de butane, 46,5% de butène, 51 % de butadiène, 1,3% de VinylAcetylène (VAC) et 0,2% de butyne. Dans certaines coupes C4, entre 0,1 et 2% poids de C3 et de C5 peut aussi être présent. Là encore les spécifications sont sévères : teneur en dioléfines strictement inférieure à 10 ppm poids pour une coupe C4 qui sera utilisée en pétrochimie ou polymérisation.

Une coupe C5 de vapocraquage présente par exemple la composition moyenne en masse suivante : 21% de pentanes, 45% de pentènes, 34% de pentadiènes.

Le procédé d'hydrogénation sélective s'est progressivement imposé pour éliminer les composés polyinsaturés des coupes pétrolières C3 à C5 citées car ce procédé permet la conversion des composés les plus insaturés vers les alcènes correspondants en évitant la saturation totale et donc la formation des alcanes correspondant.

L'hydrogénation sélective peut être réalisée en phase gaz ou liquide, de préférence en phase liquide. En effet, une réaction en phase liquide permet d'abaisser le coût énergétique et d'augmenter la durée de cycle des catalyseurs.

Pour une réaction en phase liquide, la pression est généralement comprise entre 1 et 3 MPa, la température entre 2 et 50°C et le ratio molaire hydrogène/(composés polyinsaturés à hydrogéner) entre 0,1 et 4, de préférence entre 1 et 2.

Pour une réaction d'hydrogénation en phase gazeuse, la pression est généralement comprise entre 1 et 3 MPa, la température entre 40 et 120°C et le ratio molaire hydrogène/(composés polyinsaturés à hydrogéner) entre 0,1 et 4, de préférence entre 1 et 2.

### Hydrogénation des essences de vapocraquage

Le vapocraquage produit principalement de l'éthylène, du propylène, une coupe C4 ainsi que de l'essence de vapocraquage appelée aussi essence de pyrolyse.

Selon un autre mode préféré, la charge est une essence de pyrolyse. L'essence de pyrolyse correspond à une coupe dont la température d'ébullition est généralement comprise entre 0°C et 250°C, de préférence entre 10°C et 220°C. Cette charge comprend généralement la coupe C5-C12 avec des traces de C3, C4, C13, C14, C15 (par exemple entre 0,1 à 3% poids pour chacune de ces coupes).

Par exemple, une coupe C5-200°C a généralement une composition en % poids suivante:

| | |
|---|---|
| Paraffines | 8 - 12 |
| Aromatiques | 58 - 62 |
| Mono-oléfines | 8 - 10 |
| Dioléfines | 18 - 22 |
| Soufre | 20 - 300 ppm |

L'hydrogénation sélective d'une essence de pyrolyse consiste à mettre en contact la charge à traiter avec de l'hydrogène introduit en excès dans un ou plusieurs réacteurs contenant le catalyseur d'hydrogénation.

Le débit d'hydrogène est ajusté afin d'en disposer en quantité suffisante pour hydrogéner théoriquement l'ensemble des dioléfines, des acétyléniques et des alkényl aromatiques et de maintenir un excès d'hydrogène en sortie de réacteur. Afin de limiter le gradient de température dans le réacteur, il peut être avantageux de recycler une fraction de l'effluent à l'entrée et/ou au milieu du réacteur.

Dans le cas d'une hydrogénation sélective d'essence de pyrolyse, le ratio molaire hydrogène/(composés polyinsaturés à hydrogéner) est généralement compris entre 1 et 2, la température est généralement comprise entre 40°C et 200°C, de préférence entre 50 et 180°C, la vitesse horaire spatiale (correspondant au volume d'hydrocarbure par volume de catalyseur et par heure) est comprise généralement entre 0,5 h⁻¹ et 10 h⁻¹, de préférence entre 1 h⁻¹ et 5 h⁻¹ et la pression est généralement comprise entre 1,0 MPa et 6,5 MPa, de préférence entre 2,0 MPa et 3,5 MPa.

### EXEMPLES

### Exemple 1 : Catalyseur A (selon l'invention)

Le support du catalyseur A est préparé selon la première variante du mode de préparation du support. Les étapes de préparation du support du catalyseur A se présentant sous forme de billes sont les suivantes:
s1) déshydratation par calcination flash d'hydrargillite afin d'obtenir une poudre d'alumine active. Un courant de gaz chaud permet d'éliminer et d'entraîner très rapidement l'eau évaporée. La température est fixée à 800°C et le temps de contact du matériau à déshydrater avec les gaz est de 1 seconde. La poudre d'alumine active obtenue est broyée puis est lavée avec de l'eau.
s2) mise en forme de ladite poudre d'alumine active de manière à obtenir des billes d'une densité de remplissage en cru de 785 kg/m3 et d'un diamètre compris majoritairement entre 2 et 4 mm.
   La mise en forme de ladite poudre d'alumine active de manière à obtenir des billes, nommée granulation, est réalisée au moyen d'un drageoir tournant.
s3) traitement thermique à 720°C desdites billes de manière à leur procurer une surface spécifique de 200 m²/g.
s4) traitement hydrothermal desdites billes par imprégnation avec une solution aqueuse acide. Le traitement hydrothermal est conduit à une température de 200°C durant 6,5 heures, dans un autoclave à panier rotatif, et la solution d'imprégnation est une solution aqueuse acide comprenant du nitrate d'aluminium. Ces pourcentages sont calculés en masse par rapport à la masse d'alumine introduite.
s5) calcination des agglomérés ainsi obtenus à une température de 650°C pendant 2 heures. Les agglomérés obtenus présentent une surface spécifique de 142 m²/g.

Une solution aqueuse de nitrate de palladium Pd(NO₃)₂ est préparée à 25°C par dilution de 7,5 g d'une solution aqueuse de nitrate de palladium à 10% poids et 10% poids d'acide nitrique (Aldrich) dans de l'eau déminéralisée à un volume qui correspond au volume poreux du support alumine. Cette solution est ensuite imprégnée sur 100 grammes d'un support préparé. La signature DRX de ce support est la suivante :

| Distances interréticulaires d (10⁻¹⁰ m) +/- 5.10⁻³d | Intensités relatives I/Io (en %) | Largeur de raie |
|---|---|---|
| 4,52 | 7 | L |
| 2,74 | 20 | TL |
| 2,41 | 41 | L |
| 2,28 | 22 | F |
| 2,09 | 4 | N |
| 1,993 | 47 | N |
| 1,953 | 34 | N |
| 1,651 | 4 | L |
| 1,517 | 17 | TL |
| 1,394 | 100 | N |

| | | |
|---|---|---|
| Taux d'asymétrie de la raie à 1,394Ǻ : 1,63 Rapport intensité I_{1,99}/I_{1,95} : 1,38 | | |

Le catalyseur A obtenu est séché sous air à 120°C, puis est calciné pendant 2 heures à 450°C sous air. Le catalyseur A contient 0,3 % poids de palladium.

### Exemple 2 : Catalyseur B (selon l'invention)

Le support du catalyseur B est préparé selon la première variante de mode de préparation du support. Les conditions opératoires et la méthode de préparation du support sont les mêmes que celles mises en oeuvre dans l'exemple 1.

Une solution aqueuse de nitrate de palladium Pd(NO₃)₂ est préparée à 25°C par dilution de 7,5 g d'une solution aqueuse de nitrate de palladium à 10% poids et 10% poids d'acide nitrique (Aldrich) dans de l'eau déminéralisée à un volume qui correspond au volume poreux du support alumine moins le volume de soude ajouté ensuite. A cette solution est ensuite ajouté 9,9 mL d'une solution de soude à 1 M (Aldrich) goutte à goutte sous agitation. Cette solution est ensuite imprégnée sur 100 grammes du support préparé. La signature DRX du support est la suivante :

| Distances interréticulaires d (10⁻¹⁰ m) +/- 5.10⁻³d | Intensités relatives I/Io (en %) | Largeur de raie |
|---|---|---|
| 4,53 | 6 | TL |
| 2,71 | 23 | TL |
| 2,41 | 39 | L |
| 2,28 | 25 | F |
| 2,10 | 6 | L |
| 1,993 | 51 | N |
| 1,952 | 40 | N |
| 1,647 | 4 | L |
| 1,522 | 16 | TL |
| 1,393 | 100 | N |

| | | |
|---|---|---|
| Taux d'asymétrie de la raie à 1,394Ǻ : 1,70 Rapport intensité I_{1,99}/I_{1,95} : 1,28 | | |

Le catalyseur B obtenu est séché sous air à 120°C, puis est calciné pendant 2 heures à 450°C sous air. Le catalyseur B contient 0,3 % poids de palladium.

### Exemple 3 : Catalyseur C (non conforme)

Le support du catalyseur C est préparé selon la première variante du mode de préparation du support. Les conditions opératoires et la méthode de préparation du support sont les mêmes que celles mises en oeuvre dans l'exemple1 hormis la calcination de l'étape s5) qui est réalisée à 950°C pendant 2 heures. Elle procure aux agglomérés une surface spécifique de 67 m²/g.

Une solution aqueuse de nitrate de palladium Pd(NO₃)₂ est préparée à 25°C par dilution de 7,5 g d'une solution aqueuse de nitrate de palladium à 10% poids et 10% poids d'acide nitrique (Aldrich) dans de l'eau déminéralisée à un volume qui correspond au volume poreux du support alumine. Cette solution est ensuite imprégnée sur 100 grammes du support préparé. La signature DRX du support est la suivante :

| Distances interréticulaires d (10⁻¹⁰ m) +/- 5.10⁻³d | Intensités relatives I/Io (en %) | Largeur de raie |
|---|---|---|
| 5,18 | 4 | N-L |
| 4,58 | 7 | N |
| 4,09 | 2 | N |
| 2,82 | 17 | N |
| 2,73 | 36 | F |
| 2,44 | 34 | N |
| 2,29 | 33 | N |
| 1,997 | 77 | N |
| 1,942 | 39 | N |
| 1,796 | 7 | N |
| 1,539 | 8 | N |
| 1,511 | 8 | N |
| 1,391 | 100 | N |

| | | |
|---|---|---|
| Taux d'asymétrie de la raie à 1,391Ǻ : 2,54 Rapport intensité I_{1,99}/I_{1,95} : 1,97 | | |

Le catalyseur C obtenu est séché sous air à 120°C, puis est calciné pendant 2 heures à 450°C sous air. Le catalyseur B contient 0,3 % poids de palladium.

### Exemple 4: Catalyseur D (non conforme)

Le support du catalyseur D est préparé selon la première variante du mode de préparation du support. Les conditions opératoires et la méthode de préparation du support sont les mêmes que celles mises en oeuvre dans l'exemple 1 hormis la calcination de l'étape s5) qui est réalisée à 950°C pendant 2 heures. Elle procure aux agglomérés une surface spécifique de 67 m2/g.

Une solution aqueuse de nitrate de palladium Pd(NO₃)₂ est préparée à 25°C par dilution de 7,5 g d'une solution aqueuse de nitrate de palladium à 10% poids et 10% poids d'acide nitrique (Aldrich) dans de l'eau déminéralisée à un volume qui correspond au volume poreux du support alumine moins le volume de soude ajouté ensuite. A cette solution est ensuite ajouté 9,9 mL d'une solution de soude à 1 M (Aldrich) goutte à goutte sous agitation. Cette solution est ensuite imprégnée sur 100 grammes du support préparé. La signature DRX du support est la suivante :

| Distances interréticulaires d (10⁻¹⁰ m) +/- 5.10⁻³d | Intensités relatives I/Io (en %) | Largeur de raie |
|---|---|---|
| 5,18 | 3 | N-L |
| 4,58 | 4 | L |
| 4,09 | 1 | N |
| 2,81 | 14 | L |
| 2,73 | 17 | F |
| 2,43 | 35 | L |
| 2,29 | 31 | N |
| 1,994 | 73 | N |
| 1,948 | 38 | N |
| 1,787 | 5 | L |
| 1,535 | 6 | N |
| 1,510 | 6 | N |
| 1,391 | 100 | N |

| | | |
|---|---|---|
| Taux d'asymétrie de la raie à 1,391Ǻ : 2,78 Rapport intensité I_{1,99}/I_{1,95} : 1,92 | | |

Le catalyseur D obtenu est séché sous air à 120°C, puis est calciné pendant 2 heures à 450°C sous air. Le catalyseur B contient 0,3 % poids de palladium.

### Exemple 5 : Test catalytique en hydrogénation d'un mélange styrène isoprène en présence de S.

Avant le test catalytique, les catalyseurs A, B, C et D sont traités sous un flux de 1 litre d'hydrogène par heure et par gramme de catalyseur avec une montée en température de 300°C/h et un palier à 150°C pendant 2 heures.

Les catalyseurs sont ensuite soumis à un test d'hydrogénation dans un réacteur discontinu parfaitement agité de type « Grignard ». Pour ce faire, 4 ml de billes de catalyseur réduit sont fixées à l'abri de l'air dans un panier annulaire situé autour du mobile d'agitation. Les paniers utilisés dans les réacteurs sont de type Robinson Mahonnay.

L'hydrogénation est réalisée en phase liquide.

La composition de la charge est la suivante : 8% poids styrène, 8% poids isoprène, 10 ppm de S introduits sous forme de pentanethiol, 100 ppm de S introduits sous forme de thiophène, le solvant étant du n-heptane.

Le test est réalisé sous une pression constante de 3,5 MPa d'hydrogène et à une température de 45°C. Les produits de la réaction sont analysés par chromatographie en phase gazeuse.

Les activités catalytiques sont exprimées en moles de H₂ consommées par minute et par gramme de palladium et sont reportées dans le tableau 1.

**Tableau 1: Activités mesurées en hydrogénation d'un mélange styrène-isoprène en présence de soufre**

| Catalyseur | Activité* |
|---|---|
| Catalyseur A (conforme) | 0,42 |
| Catalyseur B (conforme) | 0,51 |
| Catalyseur C (non conforme) | 0,36 |
| Catalyseur D (non conforme) | 0,44 |

| | |
|---|---|
| * en (moles H₂)/[min×(gramme de palladium)] | |

Le catalyseur A conforme à l'invention est environ 17% plus actif que le catalyseur C non conforme à l'invention pour une étape d'imprégnation identique. Le catalyseur B conforme à l'invention est environ 16% plus actifs que le catalyseur D non conforme à l'invention pour une étape d'imprégnation identique.

## Revendications

1. Catalyseur comprenant du palladium sur un support oxyde d'aluminium, ledit support d'oxyde d'aluminium présentant à l'état calciné un diffractogramme obtenu par diffractométrie de rayons X comprenant des raies qui correspondent aux distances interréticulaires d et aux intensités relatives I/Io suivantes :
| Distances interréticulaires d (10⁻¹⁰ m) +/- 5.10⁻³d | Intensités relatives I/Io (en %) |
|---|---|
| 4,54 | 3-10 |
| 2,70-2,75 | 5-25 |
| 2,41 | 35-45 |
| 2,28 | 15-30 |
| 2,10 | 0-10 |
| 1,987 | 30-50 |
| 1,958 | 30-50 |
| 1,642 | 0-5 |
| 1,519 | 10-20 |
| 1,394 | 100 |
et dans lequel la teneur en palladium dans le catalyseur est comprise entre 0,01 et 2 % poids et la surface spécifique du support est comprise entre 100 et 160 m²/g.

2. Catalyseur selon la revendication 1 dans lequel le support d'oxyde d'aluminium présente à l'état calciné un diffractogramme comprenant des raies qui correspondent aux distances interréticulaires, aux intensités relatives et aux largeurs de raie suivantes :
| Distances interréticulaires d (10⁻¹⁰ m) +/- 5.10⁻³d | Intensités relatives I/Io (en %) | Largeur de raie |
|---|---|---|
| 4,54 | 3-10 | L-TL |
| 2,70-2,75 | 5-25 | TL |
| 2,41 | 35-45 | L |
| 2,28 | 15-30 | F |
| 2,10 | 0-10 | N-TL |
| 1,987 | 30-50 | N |
| 1,958 | 30-50 | N |
| 1,642 | 0-5 | L-TL |
| 1,519 | 10-20 | TL |
| 1,394 | 100 | N |

3. Catalyseur selon l'une des revendications 1 à 2 dans lequel le diffractogramme du catalyseur sous forme oxyde comprend, en plus des raies caractéristiques du support, les raies caractéristiques du palladium, sous forme oxyde, à des distances interréticulaires d (exprimée en 10⁻¹⁰m) de : 2,67, 2,65, 2,15, 1,676,1,535, 1,523.

4. Catalyseur selon l'une des revendications 1 à 3 dans lequel le diffractogramme du catalyseur sous forme réduite et passivée à l'air comprend, en plus des raies caractéristiques du support, les raies caractéristiques du palladium sous forme réduite, à des distances interréticulaires d (en 10⁻¹⁰m) de : 2,25, 1,945, 1,375.

5. Catalyseur selon l'une des revendications 1 à 4 dans lequel le rapport des intensités relatives aux distances interréticulaires respectives de 1,987 .10⁻¹⁰ m et 1,958 .10⁻¹⁰ m est tel que (I/I₀)_{1,987}/(I/I₀)_{1,958} est compris entre 1,1 et 1,8.

6. Catalyseur selon l'une des revendications 1 à 5 dans lequel le taux d'asymétrie de la raie située à 1,394 .10⁻¹⁰ m est compris entre 1,35 et 2,0.

7. Procédé d'hydrogénation sélective dans lequel le catalyseur selon l'une des revendications 1 à 6 est mis en contact avec une charge sélectionnée dans le groupe constitué par les coupes C3 de vapocraquage, les coupes C4 de vapocraquage, les coupes C5 de vapocraquage et les essences de vapocraquage.

## Patentansprüche

1. Katalysator, umfassend Palladium auf einem Aluminiumoxidträger, wobei der Aluminiumoxidträger im kalzinierten Zustand ein Diffraktogramm aufweist, das durch Röntgenstrahldiffraktometrie erhalten ist, das Linien aufweist, die den folgenden Interplanarabständen d und den folgenden relativen Intensitäten I/Io entsprechen:
| Interplanarabstände d (10⁻¹⁰ m) +/- 5.10⁻³ d | Relative Intensitäten I/Io (in %) |
|---|---|
| 4,54 | 3-10 |
| 2,70-2,75 | 5-25 |
| 2,41 | 35-45 |
| 2,28 | 15-30 |
| 2,10 | 0-10 |
| 1,987 | 30-50 |
| 1,958 | 30-50 |
| 1,642 | 0-5 |
| 1,519 | 10-20 |
| 1,394 | 100 |
und wobei der Palladiumgehalt in dem Katalysator zwischen 0,01 und 2 Gew.% beträgt und die spezifische Oberfläche des Trägers zwischen 100 und 160 m²/g beträgt.

2. Katalysator nach Anspruch 1, wobei der Aluminiumoxidträger im kalzinierten Zustand ein Diffraktogramm aufweist, das Linien aufweist, die den folgenden Interplanarabständen, den folgenden relativen Intensitäten und den folgenden Linienbreiten entsprechen:
| Interplanarabstände D (10⁻¹⁰ m) +/ 5.10⁻³ d | Relative Intensitäten I/Io (in %) | Linienbreite |
|---|---|---|
| 4,54 | 3-10 | L-TL |
| 2,70-2,75 | 5-25 | TL |
| 2,41 | 35-45 | L |
| 2,28 | 15-30 | F |
| 2,10 | 0-10 | N-TL |
| 1,987 | 30-50 | N |
| 1,958 | 30-50 | N |
| 1,642 | 0-5 | L-TL |
| 1,519 | 10-20 | TL |
| 1,394 | 100 | N |

3. Katalysator nach einem der Ansprüche 1 bis 2, wobei das Diffraktogramm des Katalysators in Oxidform zusätzlich zu den charakteristischen Linien des Trägers die charakteristischen Linien von Palladium in Oxidform in Interplanarabständen d (ausgedrückt in 10⁻¹⁰ m) von: 2,67, 2,65, 2,15, 1,676,1,535, 1,523 aufweist.

4. Katalysator nach einem der Ansprüche 1 bis 3, wobei das Diffraktogramm des Katalysators in reduzierter und an der Luft passivierter Form zusätzlich zu den charakteristischen Linien des Trägers die charakteristischen Linien von Palladium in reduzierter Form in Interplanarabständen d (ausgedrückt in 10⁻¹⁰ m,) von: 2,25, 1,945, 1,375 aufweist.

5. Katalysator nach einem der Ansprüche 1 bis 4, wobei das Verhältnis der relativen Intensitäten zu den jeweiligen Interplanarabständen von 1,987 .10⁻¹⁰ m und 1,958 .10⁻¹⁰ m derart ist, dass (I/I₀)_{1,987}/(I/I₀)_{1,958} zwischen 1,1 und 1,8 beträgt.

6. Katalysator nach einem der Ansprüche 1 bis 5, wobei die Asymmetrierate der Linie, die bei 1,394.10⁻¹⁰ m angeordnet ist, zwischen 1,35 und 2,0 beträgt.

7. Verfahren zur selektiven Hydrierung, wobei der Katalysator nach einem der Ansprüche 1 bis 6 mit einem Füllstoff in Kontakt gebracht wird, der aus der Gruppe ausgewählt wird, die aus den C3-Schnitten aus dem Dampfcracken, den C4-Schnitten aus dem Dampfcracken, den C5-Schnitten aus dem Dampfcracken und den Dampfcrackbenzinen gebildet ist.

## Claims

1. A catalyst comprising palladium on an oxide of aluminium support, the oxide of aluminium support having, in the calcined state, a diffractogram obtained by X ray diffraction comprising peaks which correspond to the following interplanar spacings, d, and relative intensities, I/I₀:
| Interplanar spacings d (10⁻¹⁰ m) ± 5 x 10⁻³ d | Relative intensities I/Io (%) |
|---|---|
| 4.54 | 3-10 |
| 2.70-2.75 | 5-25 |
| 2.41 | 35-45 |
| 2.28 | 15-30 |
| 2.10 | 0-10 |
| 1.987 | 30-50 |
| 1.958 | 30-50 |
| 1.642 | 0-5 |
| 1.519 | 10-20 |
| 1.394 | 100 |
in which the palladium content in the catalyst is in the range 0.01% to 2% by weight and the specific surface area of the support is in the range 100 to 160 m²/g.

2. A catalyst according to claim 1, in which the oxide of aluminium support has, in the calcined state, a diffractogram comprising peaks which correspond to the following interplanar spacings and relative intensities:
| Interplanar spacings d (10⁻¹⁰ m) ± 5 x 10⁻³ d | Relative intensities I/I₀ (%) | Peak width |
|---|---|---|
| 4.54 | 3-10 | B-VB |
| 2.70-2.75 | 5-25 | VB |
| 2.41 | 35-45 | B |
| 2.28 | 15-30 | F |
| 2.10 | 0-10 | N-VB |
| 1.987 | 30-50 | N |
| 1.958 | 30-50 | N |
| 1.642 | 0-5 | B-VB |
| 1.519 | 10-20 | VB |
| 1.394 | 100 | N |

3. A catalyst according to one of claims 1 to 2, in which the diffractogram of the catalyst in the oxide form comprises, in addition to the characteristic peaks of the support, the characteristic peaks of palladium in the oxide form at interplanar spacings d (expressed in 10⁻¹⁰ m) of: 2.67, 2.65, 2.15, 1.676, 1.535, 1.523.

4. A catalyst according to one of claims 1 to 3, in which the diffractogram of the catalyst in the form which is reduced and passivated in air comprises, in addition to the characteristic peaks of the support, the characteristic peaks of palladium in the reduced form at interplanar spacings d (expressed in 10⁻¹⁰ m) of: 2.25, 1.945, 1.375.

5. A catalyst according to one of claims 1 to 4, in which the ratio of the relative intensities at respective interplanar spacings of 1.987 x 10⁻¹⁰ m and 1.958 x 10⁻¹⁰ m is such that (I/I₀)_{1.987}/(I/I₀)_{1.958} is in the range 1.1 to 1.8.

6. A catalyst according to one of claims 1 to 5, in which the asymmetry ratio of the peak located at 1.394 x 10⁻¹⁰ m is in the range 1.35 to 2.0.

7. A process for selective hydrogenation, in which the catalyst in accordance with one of claims 1 to 6 is brought into contact with a feed selected from the group constituted by steam cracking C₃ cuts, steam cracking C₄ cuts, steam cracking C₅ cuts and steam cracked gasoline.
